Europäisches Patentamt

European Patent Office

Office européen des brevets

(19)

(11) Publication number: **0 064 858**
**A1**

# EUROPEAN PATENT APPLICATION

(12)

(21) Application number: 82302285.0

(22) Date of filing: 05.05.82

(51) Int. Cl.³: **A 61 M 5/315**
**A 61 M 5/31**

(30) Priority: 07.05.81 US 261326

(43) Date of publication of application:
17.11.82 Bulletin 82/46

(84) Designated Contracting States:
BE DE FR GB NL

(71) Applicant: MERCK & CO. INC.
126, East Lincoln Avenue P.O. Box 2000
Rahway, New Jersey 07065(US)

(72) Inventor: Siman, David W.
1 Exeter Road
Clark New Jersey 07066(US)

(74) Representative: Purvis, William Michael
Cameron et al,
D. Young & Co. 10 Staple Inn
London WC1V 7RD(GB)

(54) Dose adjustable delivery system for semi-solid medicaments.

(57) A piston driven dispenser having a lockable limiting
means to restrict piston travel and thereby control the
quantity of contents delivered comprises a flanged limiting
member circumferentially mounted on a piston rod having
interrupted grooves.

Fig. 2.

Croydon Printing Company Ltd.

- 1 -                          16555

TITLE OF THE INVENTION
        DOSE ADJUSTABLE DELIVERY SYSTEM FOR
SEMI-SOLID MEDICAMENTS.


BACKGROUND OF THE INVENTION
        The present invention relates to a dispenser
for plastic substances.  More particularly it relates
to a delivery device for delivering a measured
incremental dose of a semi-solid gel or paste
medicament.
        In the past it was common to employ various
devices that delivered incremental quantities of such
substances.  Unfortunately, the means employed to
regulate the quantity delivered were either difficult
to adjust, or once adjusted, were easily dislodged
from the correct adjustment.  Furthermore, the means
to perform the adjustment were often cumbersome and
time consuming.  One such prior art device consisted
of a continuously threaded plunger having a retaining
ring threaded thereon that could be rotated to the
desired point on the plunger and thereby prevent the plunger from fully entering the medicament housing. Unfortunately, the ring was free to rotate as no way to lock the retaining ring in place was provided. Thus, a positive control over the positioning of the retaining ring was consequently also absent.

## SUMMARY OF THE INVENTION

A dispenser for regulating the delivered quantity of a substance in a semi-solid form comprises a housing for the substance and a piston for expelling it from the housing. The piston is impelled into the housing by a rod having interrupted grooves therein. A flanged stop member circumferentially mounted on said grooved rod can be locked on said rod by having the flanges seat and lock in a groove of the rod. The stop member can be disengaged from a locked position by turning it slightly about the longitudinal axis of the piston rod until the flange is free of the groove. When so disengaged, it can be freely moved along the rod and when again rotated to engage the flange, lock and thereby act as a stop means to restrict the distance the rod can travel. Thus, the distance the piston penetrates into the housing can be limited at predetermined intermediate points to thereby regulate the quantity delivered.

## DESCRIPTION OF THE DRAWINGS

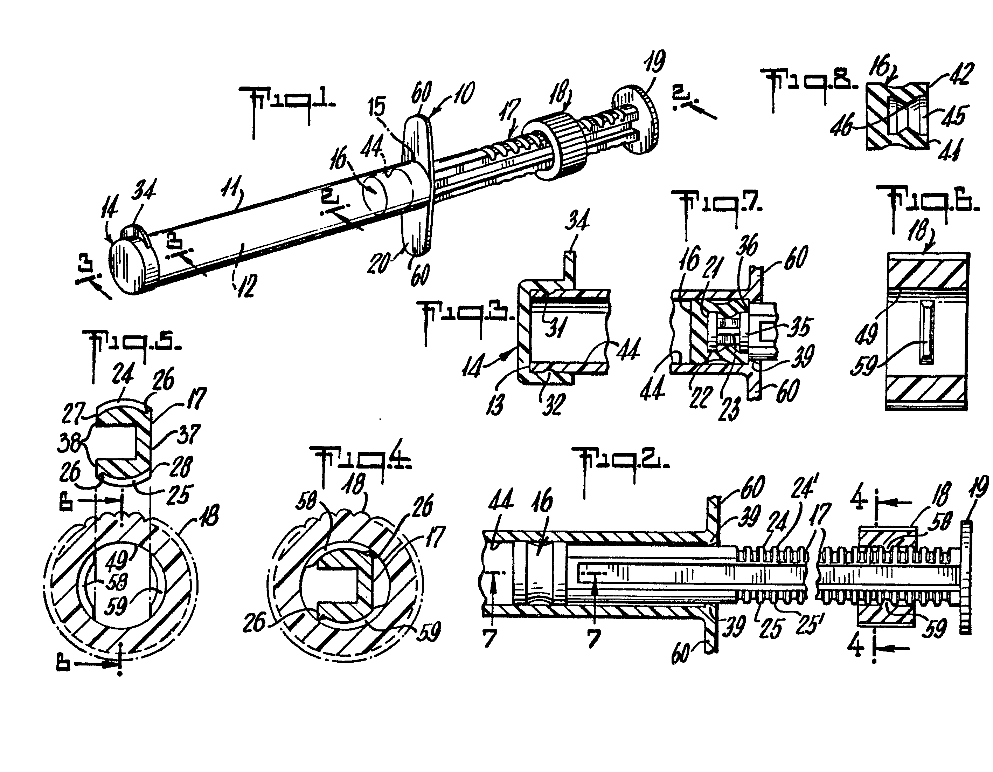

In FIG. 1 there is shown a delivery device of this invention; FIG. 2 is a cross-sectional view of the piston entering the medicament containment housing along 2-2 of FIG. 1; FIG. 3 shows a cross-sectional view of a protective cap over the end of the medicament containment housing along 3-3 of FIG. 1; FIG. 4 is a cross-sectional view through 4-4 of FIG. 2 showing the stop collar engaging the partial grooves of the piston rods; FIG. 5 is an exploded view of the stop collar and the grooved piston rod; FIG. 6 is a cross-sectional view through 6-6 of FIG. 5; FIG. 7 is a cross-sectional view through 7-7 of FIG. 2 showing the piston engaging hub on the end of the piston rod; and FIG. 8 is a partially cut away view of the piston.

## DESCRIPTION OF THE PREFERRED EMBODIMENTS

Referring now to the drawing and especially FIG. 1 there is shown a delivery device 10 having a medicament housing 11. Housing 11 is a tubular barrel having an opening at one end 13 which except during use is ordinarily capped by protective cap 14, so as to help keep the contents medicament 12 in housing 11 free from contamination. There is an opening 15 at the other end of housing 11 through which piston 16 can be inserted. Piston 16 is mounted on the proximal end of piston rod 17, and is in sealing engagement with the inner walls of medicament housing 11. At the distal end of piston rod 17 is a convenient pommel means 19 to manually slide rod 17 into or out of the barrel of housing 11, along the longitudinal axis of the housing.

The barrel of housing 11 can assume any cross-sectional shape but generally it is most convenient that the interior wall 44 have a circular cross-sectional shape. When the barrel interior is circular, of course piston 16 also assumes a circular outer edge to sealingly engage the interior wall 44 of the barrel of housing 11. Stop collar 18 is slideably mounted on rod 17, and limits the depth of longitudinal penetration of rod 17 into the interior of housing 11.

Referring now to FIGS. 2 and 7, there is shown housing 11 and piston 16, which for the sake of understanding is shown removed from the end of rod 17. At the end of rod 17, proximal to the piston, there is shown hub 21 which has circular prongs 22 and 35 spaced apart from each other to define therebetween a shoulder 23. As shown in FIG. 8, piston 16 has a hollow concavity 45 which is circumferentially surrounded by lip 46. Lip 46 is spaced from the front of piston 16 so that it engages shoulder 23 and holds prong 22 within concavity 45, and in turn holds piston 16 on hub 21. This arrangement holds piston 16 in firm but disengageable attachment to rod 17. Referring to FIG. 2, at end 15 of housing 11 there is a rim 39 about the barrel such that when piston 16 is pulled to the distal end 15 of the medicament housing 12 the periphery 42 about its back 41 will contact rim 39 and cause piston 16 to be retained within the barrel of housing 11, becoming disengaged from hub 21 as shown in FIG. 2 if piston rod 17 continues to be withdrawn. This disengaging feature seals the housing 11 so that medicament 12 can be ejected only from end 13 and not end 15 even in the event rod 17 is drawn too far out of the medicament housing 11.

This prevents the package contents from leaking and coming into any undesirable contact with the operator, as well as providing for improved insurance against inadvertant contamination or loss of package contents.

As seen best in FIG. 2, rod 17 has along its longitudinal axis a plurality of transverse grooves 24 and 25. Grooves 24 are spaced apart from one another as are grooves 25. Groove 24' has a mate, groove 25', as best shown in FIG. 5. At opposite sides of grooves 24' and 25' there is an open end 26 and a closed end 27.

Transverse grooves 24 and 25 are generally at an angle of from 30° to 90° with the longitudinal axis of rod 17. As shown in a preferred embodiment, FIG. 4, the grooves are at a 90° angle with the longitudinal axis.

Referring to FIG. 4, and 5, rod 17 is shown as having indented sides 37, 38, juxtaposed to the sides bearing grooves 24 and 25. One side, for example side 37, can have etched, molded or printed thereon a convenient legend or scale to provide for a precise positioning stop collar 18 according to the appropriate quantity to be delivered.

The stop collar 18 has an aperature 49, with arcuate opposing flanges 58, 59 partially surrounding aperature 49. The space between flanges 58 and 59 is sufficient to clear the grooves 24 and 25 when collar 18 is rotated so that flanges 58 and 59 are aligned with indented sides 37 and 38. This permits the collar to slide freely along the longitudinal axis of rod 17. Preferably there is provided opposing

0064858

flanges 58, 59 to lock in grooves 24, 25. This provides a more secure lock when the flanges are locked in the grooves. But clearly one set or three or more sets of flanges and grooves are satisfactory so long as the flanges can clear the grooves when the collar is in the unlocked position.

Prior to operation a suitable medicament 12 is loaded into housing 11. So loaded, rod 17 is positioned so that it draws piston 16 against rim 39.

For delivery, the operator selects what proportion of the medicament load is desired in a dose. Having done so, the stop collar is turned or rotated about the longitudinal axis of rod 17 until flanges 58, 59 are free from grooves 24 and 25. The stop collar 18 is then moved along rod 17 until it reaches the point on the scale of side 37 appropriate for such medicament load and is thus in juxtaposition to the desired set of grooves 24, 25. The stop collar 18 is then again rotated so that flanges 58, 59 enter open ends 27, 28 of grooves 24, 25 respectively. The stops 26, 28 at the ends of grooves 24, 25 prevent stop collar from being rotated further, and lock it in place. Once locked, the operator removes cap 14, inserts the barrel of housing 11 into the body cavity or other area where the contents are to be delivered, wraps his fingers about finger grips 60, placing his palm against pommel 19 and pushes rod 17 inward by means of pressure against pommel 19 until collar 18 abutts the grips 60 at the opening 15 in housing 11, or other appropriate means to halt the inward travel of rod 17 handle 20 limiting rod 17 from further penetration into housing 12.  This action expells a desired dose from the housing through end 13.  To proceed to deliver a subsequent dose the collar 18 is rotated so that flanges 58, 59 pass out of grooves 24, 25 by passing through open ends 27, 28 respectively.  The collar, freed of restraint, is then moved distally and again cocked in place.

Now having described my invention other embodiments will suggest themselves to others skilled in the art, which embodiments do not depart from the appended claims.

WHAT IS CLAIMED IS:

1. A dispenser for regulating the delivered quantity of a plastic substance comprising a housing having endwalls and sidewalls for containing the substance, said housing having an opening in one endwall thereof for egress of the substance; a piston in sealing engagement with the sidewalls of the housing and adapted to travel between the endwalls; an opening in the second endwall; a rod extending into the housing through the opening in the second endwall; the piston mounted on the end of the rod extending into the housing; the rod having a plurality of spaced apart grooves transverse to the longitudinal axis of the rod; said grooves extending only partially around the rod; a notable slideable collar mounted on the rod; said collar having at least one flange thereon for engaging one of the grooves whereby when a flange is engaged in a groove the penetration of the piston into the housing is limited by the collar meeting the housing; and the collar when rotated about the longitudinal axis of the rod frees said flange from the grooves for slideable positioning therealong.

2. A delivery device according to Claim 1 where the piston is detachably mounted on the rod.

3. A delivery device according to Claim 2 where there is a retaining flange about the second endwall opening adapted to sealingly engage the piston.

4.  A delivery device according to Claim 2 wherein a circumferential flange extends about the second endwall opening and is of a lesser diameter than the circumferential diameter of the piston whereby when the rod is with drawn fully from the housing said piston is retained therein.

5.  A delivery device according to Claim 1 wherein two arcuate flanges opposing each other are mounted on the inside of said collar.

6.  A delivery device according to Claim 1 wherein the groove is open at one end to receive a flange member of the collar, at the other end is closed to prevent passage of the flange beyond the closed end.

7.  A dispenser for regulating the delivered quantity of a plastic substance comprising a housing having endwalls and sidewalls for containing the substance, said housing having an opening in a first endwall for egress of the substance; a piston in sealing engagement with the sidewalls of the housing and adapted to travel between the endwalls; an opening in the second endwall; a rod extending into the housing through the an opening in the second endwall; the piston detachably mounted on the end of the rod within the housing; a retention flange extending circumferentially about the second endwall inside the housing and about the rod to prevent the piston from passage through the opening in the second endwall; the rod having a top, bottom and sides; a pair of grooves in the top and bottom of the rod; a collar circumferentially mounted about the rod having opposed arcuate flanges extending partially about the inner side of the collar, each flange of a length sufficient to pass into a groove, but sufficiently short to clear the sides of the rod; said collar larger in cross section than the opening in the second endwall; and said grooves open at one opposing end to receive the arcuate flanges, and closed at the other opposing end to prevent passage of the arcuate flanges therebeyond.

8. A device according to Claim 7 where there is a pommel mounted on the end of the rod distal the piston.

9. A device according to Claim 1 or 7 where there is a plurality of grooves equidistantly spaced on the top and bottom of the rod.

10. A device according to Claims 1, 7 or 10 wherein the grooves are marked with gradations calibrated to correspond with the quantity of substance delivered.

0064858

0064858

European Patent Office

**EUROPEAN SEARCH REPORT**

Application number

EP 82 30 2285

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | WO-A-8 101 104 (J.SILVER)<br><br>*Abstract, page 6, lines 4-38; page 7, lines 1-29; page 8, lines 14-26; page 9, lines 13-17, 29-35; page 10, lines 12-22; claims 1,7-10; figures 1,3,4* | 1,2,5, 6,9,10 | A 61 M 5/315<br>A 61 M 5/31 |
| Y | --- | 3,4,7, 8 | |
| X | GB-A-1 550 308 (PLASPHARM)<br><br>*Page 1, lines 18-62; page 2, lines 25-31; claims 1-3,5,6; figures 1,4,5* | 1,2,5, 9,10 | |
| Y | --- | 3,4,7, 8 | **TECHNICAL FIELDS SEARCHED (Int. Cl. 3)** |
| Y | US-A-3 729 032 (E.A. TISCHLINGER)<br>*Column 9, lines 11-56, 60-67; column 12, lines 27-64; figures 2,8* | 3,4,7, 8 | A 61 M |
| Y | US-A-4 026 287 (I. HALLER)<br><br>*Column 1, lines 49-57; figure 1* | 3,4,7, 8 | |
| Y | --- <br>GB-A-1 106 825 (LONG & HAMBLY)<br>*Claims; figures* | 2 | |

--- 

-/-

The present search report has been drawn up for all claims

| Place of search | Date of completion of the search | Examiner |
|---|---|---|
| THE HAGUE | 05-08-1982 | ENGELBRECHT E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0064858

**European Patent Office**

**EUROPEAN SEARCH REPORT**

Application number

EP 82 30 2285

| DOCUMENTS CONSIDERED TO BE RELEVANT | | | Page 2 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | CH-A- 419 458 (G. ZÖBL) *Claims; figure 1* | 3,4 | |
| A | GB-A- 958 636 (KONINKLIJKE NEDERLANDSCHE GIST EN SPIRITUSFABRIEK) & NL - A - 113 374 (Cat.A) | | |
| A | EP-A-0 022 987 (CUTTER LAB.) | | |
| | | | TECHNICAL FIELDS SEARCHED (Int. Cl. ³) |

The present search report has been drawn up for all claims

| Place of search | Date of completion of the search | Examiner |
|---|---|---|
| THE HAGUE | 05-08-1982 | ENGELBRECHT E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82